# EUROPEAN PATENT SPECIFICATION

(11) **EP 4 344 635 B1**
(45) Date of publication and mention of the grant of the patent: **01.07.2026**
(21) Application number: 23164212.5
(22) Date of filing: 27.03.2023
(51) Int. Cl.: A61B 5/16, A61B 5/18, A61B 5/00, B60K 28/06, B60W 40/04, B60W 40/08

(54) **DRIVER ABNORMALITY SIGN DETECTION APPARATUS**
VORRICHTUNG ZUR ERKENNUNG VON ANOMALIEN BEI EINEM FAHRER
APPAREIL DE DÉTECTION DE SIGNES D'ANOMALIE DE CONDUCTEUR

(30) Priority: 28.09.2022 JP 2022154856
(43) Date of publication of application: 03.04.2024
(73) Proprietor: MAZDA MOTOR CORPORATION, Hiroshima 730-8670 (JP)
(72) Inventor: Fukui, Akiko, Aki-gun, Hiroshima, 730-8670 (JP); Iwase, Koji, Aki-gun, Hiroshima, 730-8670 (JP); Iwashita, Yohei, Aki-gun, Hiroshima, 730-8670 (JP)
(74) Representative: Lorenz Seidler Gossel Part. mbB

(56) References cited:
- US-A1- 2009 303 078
- US-A1- 2021 316 736
- US-A1- 2022 289 151

## Description

### [Technical Field]

The present invention relates to a driver abnormality sign detection apparatus for detecting an abnormality sign state of a driver during driving of a vehicle.

### [Background Art]

Conventionally, a driver state detection apparatus that detects an abnormal state of a driver of a vehicle has been proposed (for example, see Patent document 1, JP-A-2021-077136). In the apparatus disclosed in Patent document 1, an amplitude and a frequency of the vehicle driver's saccade (jumping eye motion of the driver to intentionally move his/her eye gaze) are detected, and such an attention level is detected that is increased as the number of caution points to be checked by the driver during travel is increased in vehicle outside environment, so as to detect the abnormality of the driver on the basis of the attention level and the amplitude and the frequency of the driver's saccade.

A driver abnormality sign detection apparatus according to the preamble of claim 1 is known from documents US 2022/289151 A1 and US 2009/303078 A1.

### [Summary of the Invention]

### [Problem to be solved by the invention]

However, in a state where the driver's attention function is slightly declined due to a mild disease, aging, or the like but the driver can still drive, that is, an abnormality sign state which typically occurs prior to an abnormal state where the driver has difficulty in driving, no obvious difference appears to the amplitude and the frequency of the driver's saccade in comparison with a state with no abnormality. Thus, the abnormality sign state cannot be detected by the related art as described above.

The invention has been made to solve such a problem and therefore has a purpose of providing a driver abnormality sign detection apparatus capable of detecting an abnormality sign state of a driver at a stage sufficiently earlier than onset of an abnormal state where the driver has difficulty in driving.

### [Means for solving the Problem]

The above-described object is solved by the subject matter of claim 1. Preferred emdodiments form the subject matter of the dependent claims.

In order to solve the above-described problem, the invention is a driver abnormality sign detection apparatus that detects an abnormality sign state of a driver who drives a vehicle, and includes: a travel environment information acquisition device that acquires travel environment information of the vehicle; an eye gaze tracker (to which is sometimes referred to as sightline detector) that detects the driver's eye gaze; and a controller configured to detect the driver's abnormality sign state on the basis of the travel environment information and the driver's eye gaze (to which is sometimes referred to as driver's sightline). The controller is configured to: identify a visual recognition required point to be visually recognized by the driver on the basis of the travel environment information; determine whether the driver has visually recognized the visual recognition required point on the basis of the driver's eye gaze; and detect the driver's abnormality sign state on the basis of a fact that the driver has not visually recognized the visual recognition required point.

According to the present invention that is configured as described above, the controller identifies the visual recognition required point to be visually recognized by the driver on the basis of the travel environment information, determines whether the driver has visually recognized the visual recognition required point on the basis of the the driver's eye gaze, and detects the driver's abnormality sign state on the basis of the fact that the driver has not visually recognized the visual recognition required point. Thus, it is possible to detect the abnormality sign state by using a fact that the driver has not been able to visually recognize the visual recognition required point due to a decline in a distributive attention function or a reduction in an effective visual field at an initial stage of a decline in the driver's attention function. In this way, it is possible to detect the driver's abnormality sign state at a stage, at which no obvious difference appears to the amplitude and the frequency of the driver's saccade and which is sufficiently earlier than onset of the abnormal state where the driver has difficulty in driving, for example.

In the invention, preferably, the controller is configured to: detect the driver's abnormality sign state on the basis of the number of the visual recognition required point not visually recognized by the driver.

According to the invention that is configured as described above, the driver's abnormality sign state is detected on the basis of the number of the visual recognition required point to which the driver's eye gaze is not directed. Thus, when the driver can no longer visually recognize the plural visual recognition required points at the same time due to the decline in the distributive attention function and starts missing the visual recognition required points at the initial stage of the decline in the attention function, the abnormality sign state can be detected on the basis of the severity of missing of the visual recognition required point. In this way, it is possible to appropriately detect the driver's abnormality sign state at the stage sufficiently earlier than the onset of the abnormal state where the driver has difficulty in driving.

In the invention, preferably, the controller is configured to detect the driver's abnormality sign state in the case where a ratio of the number of the visual recognition required point not visually recognized by the driver to the total number of the visual recognition required points is equal to or larger than a specified value.

According to the invention that is configured as described above, when the driver can no longer visually recognize the plural visual recognition required points at the same time due to the decline in the distributive attention function and the missing ratio of the visual recognition required point becomes equal to or higher than the specified value at the initial stage of the decline in the attention function, the abnormality sign state can be detected. In this way, it is possible to appropriately detect the driver's abnormality sign state at the stage sufficiently earlier than the onset of the abnormal state where the driver has difficulty in driving.

In the invention, preferably, the controller is configured to: estimate a size of an effective visual field that can be visually recognized by the driver on the basis of a distance between the driver's sightline or the driver's eye gaze and the visual recognition required point not visually recognized by the driver; and detect the driver's abnormality sign state in the case where the estimated effective visual field size is smaller than the specified value.

According to the invention that is configured as described above, when the effective visual field is narrowed and the driver starts missing the visual recognition required point at the initial stage of the decline in the attention function, the abnormality sign state can be detected by estimating the degree of the narrowness of the effective visual field. In this way, it is possible to appropriately detect the driver's abnormality sign state at the stage sufficiently earlier than the onset of the abnormal state where the driver has difficulty in driving.

In the invention, preferably, the controller is configured to estimate the size of the effective visual field by comparing a minimum value of the angles of the driver's head with respect to at least one visual recognition required point that has not been visually recognized and the driver's eye gaze with a maximum value of the angles of the driver's head with respect to at least one visual recognition required point that has been visually recognized and the driver's eye gaze.

The invention, preferably, the controller is configured to estimate the size of the effective visual field by being an intermediate value, preferably a mean value, between the minimum value of the angles of the driver's head with respect to at least one visual recognition required point that has not been visually recognized and the driver's eye gaze and the maximum value of the angles of the driver's head with respect to at least one visual recognition required point that has been visually recognized and the driver's eye gaze.

In the invention, preferably, the driver abnormality sign detection apparatus further includes an information output device that outputs information to the driver. The controller is configured to cause the information output device to output information that is used to guide the driver's eye gaze to the visual recognition required point in the case where the driver's abnormality sign state is detected.

According to the invention that is configured as described above, in the case where the driver's abnormality sign state is detected, the driver's eye gaze is guided to the visual recognition required point. Therefore, it is possible to provide the appropriate driver assistance to the driver who starts missing the visual recognition required point at the initial stage of the decline in the attention function.

In the invention, preferably, the travel environment information acquisition device (21-24) for acquiring travel environment information of the vehicle (1) comprises at least one of: an outside camera (21) for capturing an image around the vehicle (1), a radar (22) for measuring a position and a speed of an object with respect to the vehicle (1), a navigation system (23) for providing map information to the controller (10), and a speed sensor for detecting a speed of the vehicle (1).

In the invention, preferably, the eye gaze tracker comprises an in-vehicle camera for capturing an image of the driver of the vehicle (1).

In the invention, the driver abnormality sign detection apparatus further comprises:
a driving operation detector (25-31) for detecting a driving operation of the vehicle (1) caused by the driver, wherein
the controller (10) is adapted to determine that the driver has visually recognized the visual recognition required point, in the case where the driver's eye gaze is directed within a specified range including the visual recognition required point, and, thereafter, the driving operation performed by the driver of the vehicle corresponds to the visual recognition required point.

In the invention, preferably, the controller (10) is adapted to determine that the driver has not visually recognized the visual recognition required point, in the case where the driver's eye gaze is directed within a specified range including the visual recognition required point, and, thereafter, the driving operation performed by the driver of the vehicle fails to correspond to the visual recognition required point.

In the invention, preferably, the driving operation detector (25-31) comprises at least one of: a vehicle speed sensor (25) for detecting a speed of the vehicle (1), a yaw rate sensor (27) for detecting a yaw rate of the vehicle (1), a steering angle sensor (28) for detecting a rotation angle of a steering wheel (5), a steering torque sensor (29) for detecting torque applied to a steering shaft via a steering wheel (5), an acceleration sensor (26, 30) for detecting an depression amount of an accelerator pedal, and a brake sensor (31) for detecting a depression amount of a brake pedal.

In the invention, preferably, the visual recognition required point is a red light or a stop sign, the driving operation corresponding thereto is the decelerating operation.

In the invention, preferably, the driver abnormality sign detection apparatus further comprises a visual recognition required point database in which the travel environment and the visual recognition required points are stored in a mutually corresponding manner, for identifying the visual recognition required points to be visually recognized by the driver on basis of the current travel environment detected by the travel environment information acquisition device.

The invention is further directed to a vehicle comprising the driver abnormality sign detection apparatus according to the invention.

### [Advantage of the invention]

According to the driver abnormality sign detection apparatus of the invention, the driver's abnormality sign state can be detected at the stage sufficiently earlier than the abnormal state where the driver has difficulty in driving.

### [Brief Description of the Drawings]

[Fig. 1] Fig. 1 is an explanatory view of a vehicle on which a driver abnormality sign detection apparatus according to an embodiment of the invention is mounted.
[Fig. 2] Fig. 2 is a block diagram of the driver abnormality sign detection apparatus according to the embodiment of the invention.
[Fig. 3] Fig. 3 is a control block diagram of abnormality sign detection according to the embodiment of the invention.
[Fig. 4] Fig. 4 is a table exemplifying information that is stored in a visual recognition required point database according to the embodiment of the invention.
[Fig. 5] Fig. 5 is a flowchart of detection processing of an abnormality sign state according to the embodiment of the invention.

### [Modes for Carrying Out the Invention]

A description will hereinafter be made on a driver abnormality sign detection apparatus according to an embodiment of the invention with reference to the accompanying drawings.

### [System Configuration]

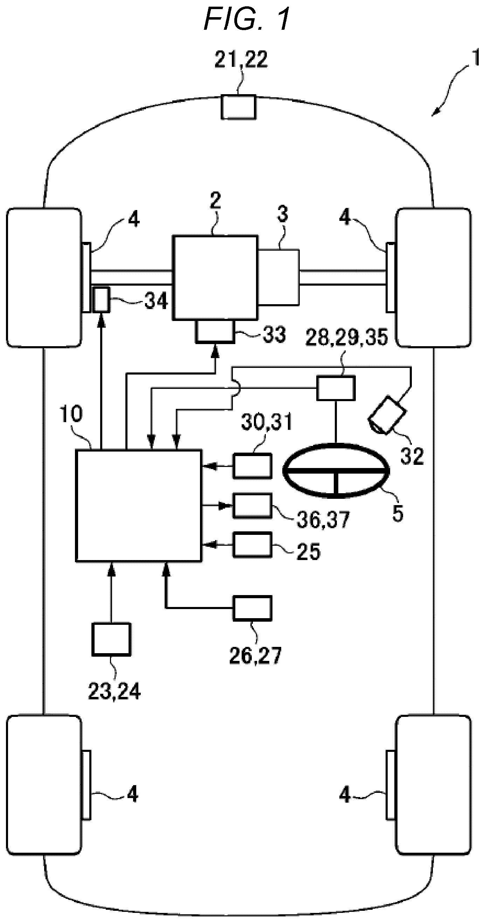

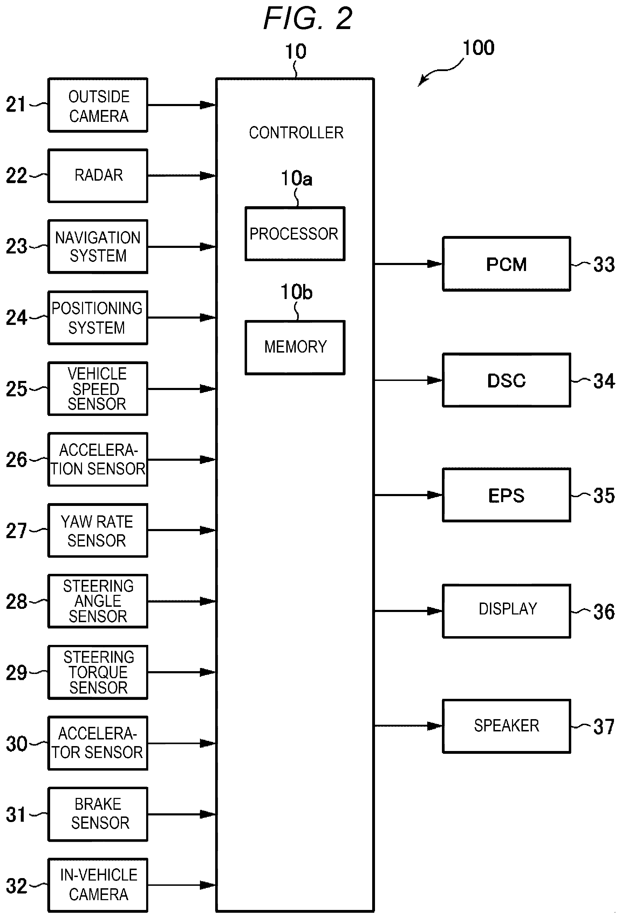

First, a description will be made on a configuration of the driver abnormality sign detection apparatus according to this embodiment with reference to Fig. 1 and Fig. 2. Fig. 1 is an explanatory view of a vehicle on which the driver abnormality sign detection apparatus is mounted, and Fig. 2 is a block diagram of the driver abnormality sign detection apparatus.

A vehicle 1 according to this embodiment includes: drive power sources 2 such as an engine and an electric motor that output drive power; a transmission 3 that transmits the drive power output from the drive power source 2 to drive wheels; a brake 4 that applies a braking force to the vehicle 1; and a steering device 5 for steering the vehicle 1.

A driver abnormality sign detection apparatus 100 is configured to detect an abnormality sign state of a driver of the vehicle 1 and to execute control of the vehicle 1 and driving assistance control when necessary. As illustrated in Fig. 2, the driver abnormality sign detection apparatus 100 has a controller 10, plural sensors, plural control systems, and plural information output devices.

More specifically, the plural sensors include an outside camera 21 and a radar 22 for acquiring travel environment information of the vehicle 1 as well as a navigation system 23 and a positioning system 24 for detecting a position of the vehicle 1. The plural sensors also include a vehicle speed sensor 25, an acceleration sensor 26, a yaw rate sensor 27, a steering angle sensor 28, a steering torque sensor 29, an acceleration sensor 30, and a brake sensor 31 for detecting behavior of the vehicle 1 and the driver's driving operation. The plural sensors further include an in-vehicle camera 32 for detecting the driver's eye gaze. The plural control systems include: a powertrain control module (PCM) 33 that controls the drive power source 2 and the transmission 3; a dynamic stability control system (DSC) 34 that controls the drive power source 2 and the brake 4; and an electric power steering system (EPS) 35 that controls the steering device 5. The plural information output devices include a display 36 that outputs image information and a speaker 37 that outputs voice information.

In addition, other sensors may include a peripheral sonar that measures a distance and a position of a peripheral structure relative to the vehicle 1, a corner radar that measures approach of the peripheral structure to four corner sections of the vehicle 1, and various sensors (a heart rate sensor, an electrocardiogram sensor, a steering wheel grip force sensor, and the like) that detect the driver's state.

The controller 10 executes various arithmetic operations on the basis of signals received from the plural sensors, transmits, to the PCM 33, the DSC 34, and the EPS 35, a control signal for appropriately actuating the drive power source 2, the transmission 3, the brake 4, and the steering device 5, and transmits, to the display 36 and the speaker 37, a control signal for outputting desired information. The controller 10 is a computer that includes one or more processors 10a (typically, a CPU), memory 10b (ROM, RAM, and the like) that stores various programs and data, an input/output device, and the like.

The outside camera 21 captures an image around the vehicle 1 and outputs image data. The controller 10 identifies an object (a preceding vehicle, a parked vehicle, a pedestrian, a travel road, a lane marking (a lane divider, a white line, or a yellow line), a traffic signal, a traffic sign, a stop line, an intersection, an obstacle, or the like) on the basis of the image data received from the outside camera 21. The outside camera 21 corresponds to an example of the "travel environment information acquisition device" in the invention.

The radar 22 measures a position and a speed of the object (particularly, the preceding vehicle, the parked vehicle, the pedestrian, a dropped object on the travel road, or the like). For example, a millimeter-wave radar can be used as the radar 22. The radar 22 transmits a radio wave in an advancing direction of the vehicle 1, and receives a reflected wave that is generated when the object reflects the transmitted wave. Then, based on the transmitted wave and the received wave, the radar 22 measures a distance between the vehicle 1 and the object (for example, an inter-vehicular distance) and a relative speed of the object to the vehicle 1. In this embodiment, instead of the radar 22, a laser radar, an ultrasonic sensor, or the like may be used to measure the distance from and the relative speed of the object. Alternatively, the plural sensors may be used to constitute a position and speed measuring device. The radar 22 corresponds to an example of the "travel environment information acquisition device" in the invention.

The navigation system 23 stores map information therein and can provide the map information to the controller 10. Based on the map information and current vehicle position information, the controller 10 identifies a road, the intersection, the traffic signal, a building, or the like that exists around (particularly, in the advancing direction of) the vehicle 1. The map information may be stored in the controller 10. The positioning system 24 is a GPS system and/or a gyroscopic system, and detects the position of the vehicle 1 (the current vehicle position information). Each of the navigation system 23 and the positioning system 24 also corresponds to an example of the "travel environment information acquisition device" in the invention.

The vehicle speed sensor 25 detects a speed of the vehicle 1 on the basis of a rotational speed of the wheel or a driveshaft, for example. The acceleration sensor 26 detects acceleration of the vehicle 1. This acceleration includes acceleration in a front-rear direction of the vehicle 1 and acceleration in a lateral direction (that is, lateral acceleration) thereof. In the present specification, the acceleration includes not only a change rate of the speed in a speed increasing direction but also a change rate of the speed in a speed reducing direction (that is, deceleration).

The yaw rate sensor 27 detects a yaw rate of the vehicle 1. The steering angle sensor 28 detects a rotation angle (a steering angle) of a steering wheel of the steering device 5. The steering torque sensor 29 detects torque (steering torque) applied to a steering shaft via the steering wheel. The acceleration sensor 30 detects a depression amount of an accelerator pedal. The brake sensor 31 detects a depression amount of a brake pedal.

The in-vehicle camera 32 captures an image of the driver and outputs image data. The controller 10 detects the driver's eye gaze direction on the basis of the image data that is received from the in-vehicle camera 32. The in-vehicle camera 32 corresponds to an example of the " eye gaze tracker" (to which is sometimes referred to as sightline detector) in the invention.

The PCM 33 controls the drive power source 2 of the vehicle 1 to adjust the drive power of the vehicle 1. For example, the PCM 33 controls an ignition plug of the engine, a fuel injection valve, a throttle valve, a variable valve mechanism, the transmission 3, an inverter that supplies electric power to the electric motor, and the like. When the vehicle 1 has to be accelerated or decelerated, the controller 10 transmits the control signal to the PCM 33 so as to adjust the drive power.

The DSC 34 controls the drive power source 2 and the brake 4 of the vehicle 1 and thereby executes deceleration control and posture control of the vehicle 1. For example, the DSC 34 controls a hydraulic pump, a valve unit, and the like of the brake 4, and controls the drive power source 2 via the PCM 33. When it is necessary to execute the deceleration control or the posture control of the vehicle 1, the controller 10 transmits the control signal to the DSC 34 so as to adjust the drive power or generate the braking force.

The EPS 35 controls the steering device 5 of the vehicle 1. For example, the EPS 35 controls the electric motor, which applies the torque to the steering shaft of the steering device 5, and the like. When the advancing direction of the vehicle 1 has to be changed, the controller 10 transmits the control signal to the EPS 35 so as to change a steering direction.

The display 36 is provided in front of the driver in a cabin and displays the image information to the driver. As the display 36, for example, a liquid-crystal display or a head-up display is used. The speaker 37 is installed in the cabin and outputs various types of the voice information.

### [Summary of Driver Abnormality Sign Detection]

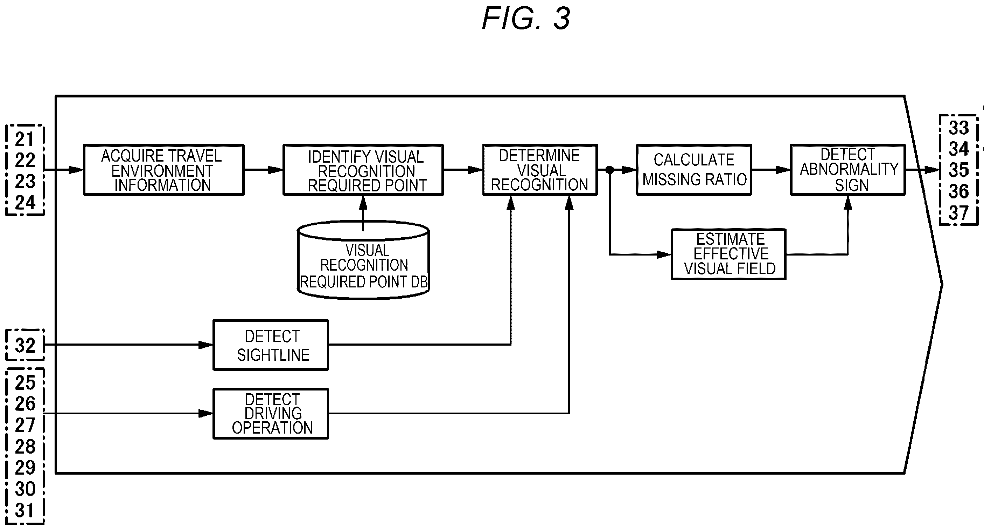

Next, a description will be made on a basic concept of driver abnormality sign detection that is executed by the above-described controller 10 in this embodiment with reference to Fig. 3. Fig. 3 is a control block diagram of the abnormality sign detection according to this embodiment.

In the abnormality sign state prior to an abnormal state where the driver has difficulty in driving, it is considered that the driver's attention function starts being declined due to a mild disease, aging, or the like. In view of the above, the present inventors considered that the abnormality sign state could be detected by detecting this decline in the driver's attention function.

The driver's attention function mainly includes: a function to see plural objects simultaneously (a distributive attention function); a function to select the object from the plural objects and see the selected object (a selective attention function); a function to switch the object (a shifting attention function); and a function to keep seeing the object (a continuous attention function). A result of research by the present inventors has led to such a finding that, in the case where the attention function is declined due to a certain disease (a cardiac disorder, a brain disorder, hypoglycemia, or the like) or aging, the attention function is declined in an order of the distributive attention function → the selective attention function → the shifting attention function → the continuous attention function. That is, at an initial stage of the decline in the attention function, plural visual recognition required points that should be recognized visually (recognized by seeing with eyes) by the driver during driving can no longer be visually recognized at the same time, and the driver begins to miss some of the visual recognition required points due to the decline in the distributive attention function. Thus, the abnormality sign state can be detected on the basis of severity of missing of the visual recognition required point.

The result of the research by the present inventors has also led to such a finding that, a patient whose attention function is declined has a narrow effective visual field (of the entire visual field, a range where information can be acquired effectively) and thus cannot visually recognize the object even when his/her eye gaze is directed to vicinity of the object. That is, at the initial stage of the decline in the attention function, since the effective visual field is narrowed, it becomes difficult to visually recognize the visual recognition required point, and consequently, the visual recognition required point is missed. Thus, the abnormality sign state can be detected by estimating a degree of narrowness of the effective visual field from a distance between the visual recognition required point and the sightline or eye gaze at the time when the visual recognition required point cannot be recognized visually.

Accordingly, the controller 10 in this embodiment is configured to identify the visual recognition required point on the basis of the travel environment information, determines whether the driver has visually recognized the visual recognition required point on the basis of the driver's sightline or the driver's eye gaze, and identifies the severity of missing of the visual recognition required point and a size of the effective visual field on the basis of a fact that the driver has not visually recognized the visual recognition required point, so as to detect the driver's abnormality sign state.

More specifically, as illustrated in Fig. 3, the controller 10 acquires the travel environment information on the basis of the signals that are received from the sensors including the outside camera 21, the radar 22, the navigation system 23, and the positioning system 24 (ACQUIRE TRAVEL ENVIRONMENT INFORMATION). The travel environment information is information on environment in which the vehicle 1 travels. Examples of the travel environment information are: type information and positional information of the object, such as the preceding vehicle, the parked vehicle, the pedestrian, the travel road, the lane marking (the lane divider, the white line, or the yellow line), the traffic signal, the traffic sign, the stop line, the intersection, or the obstacle, that exists around the vehicle 1; information on a type and a shape of the road on which the vehicle 1 travels; information on the peripheral building; and information on a current position of the vehicle 1.

Based on the acquired travel environment information, the controller 10 refers to a visual recognition required point database in which the travel environment and the visual recognition required points are stored in a mutually corresponding manner, and identifies the visual recognition required point to be visually recognized by the driver (IDENTIFY VISUAL RECOGNITION REQUIRED POINT). The visual recognition required point database is stored in the memory 10b or the like in advance.

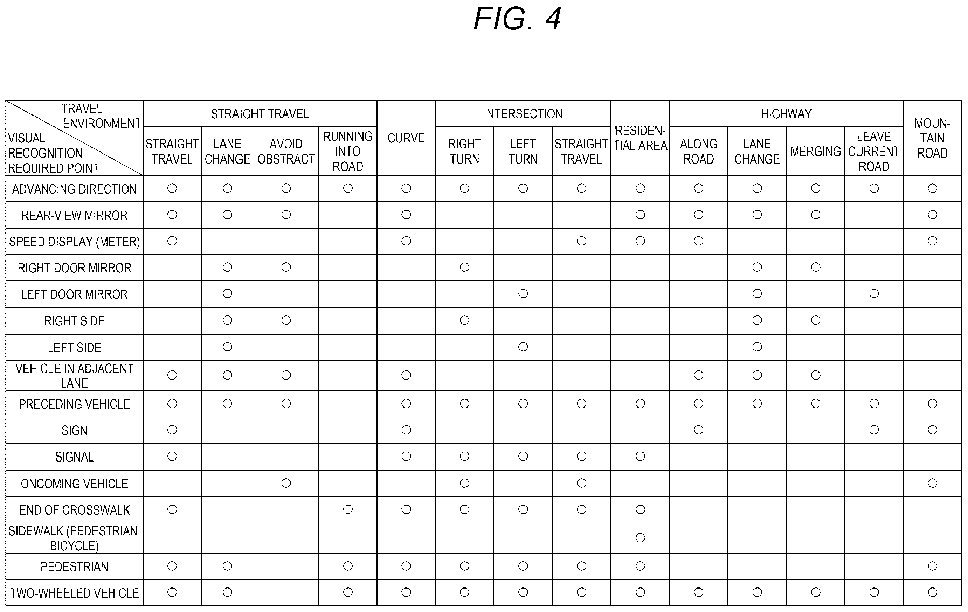

Fig. 4 is a table exemplifying information that is stored in the visual recognition required point database according to this embodiment. Each row in the visual recognition required point database exemplified in Fig. 4 represents the travel environment in which the vehicle 1 travels ("STRAIGHT TRAVEL" in "STRAIGHT TRAVEL", "CURVE", and the like). Meanwhile, each column in the visual recognition required point database exemplified in Fig. 4 represents the visual recognition required point to be visually recognized by the driver ("ADVANCING DIRECTION", "REAR-VIEW MIRROR", and the like). Then, the visual recognition required point to be visually recognized by the driver in each of the travel environments is circled. The controller 10 identifies the travel environment of the vehicle 1 on the basis of the acquired travel environment information, and acquires the visual recognition required point in the identified travel environment from the visual recognition required point database. For example, in the case where the travel environment is "LEFT TURN" under "INTERSECTION", in the example illustrated in Fig. 4, the visual recognition required points are identified as "ADVANCING DIRECTION", "LEFT DOOR MIRROR", "LEFT SIDE", "PRECEDING VEHICLE", "SIGNAL", "END OF CROSSWALK", "PEDESTRIAN", and "TWO-WHEELED VEHICLE".

The controller 10 detects the driver's eye gaze on the basis of the signal received from the in-vehicle camera 32 (DETECT SIGHTLINE). Then, the controller 10 determines whether the driver has visually recognized the visual recognition required point on the basis of the identified visual recognition required point and the detected driver's eye gaze (DETERMINE VISUAL RECOGNITION). More specifically, in the case where the driver's sightline or eye gaze is directed within a specified range including the visual recognition required point (for example, in the case where an angle defined by a direction from the driver's head toward the visual recognition required point and the driver's eye gaze falls within five degrees), the controller 10 determines that the driver has visually recognized the visual recognition required point. This specified range is set and stored in the memory 10b or the like in advance.

The controller 10 detects the driver's driving operation on the basis of the signals that are received from the sensors including the vehicle speed sensor 25, the acceleration sensor 26, the yaw rate sensor 27, the steering angle sensor 28, the steering torque sensor 29, the acceleration sensor 30, and the brake sensor 31 (DETECT DRIVING OPERATION). Examples of the driving operation are an accelerating operation, a decelerating operation, a left turn operation, and a right turn operation.

Then, the controller 10 determines whether the driver has visually recognized the visual recognition required point on the basis of the identified visual recognition required point as well as the detected driver's eye gaze and driving operation (DETERMINE VISUAL RECOGNITION). More specifically, in the case where the driver's eye gaze is directed within the specified range including the visual recognition required point (for example, in the case where the angle defined by the direction from the driver's head toward the visual recognition required point and the driver's eye gaze falls within 30 degrees), and the driving operation corresponding to the visual recognition required point is thereafter performed, the controller 10 determines that the driver has visually recognized the visual recognition required point. This specified range is set and stored in the memory 10b or the like in advance. Here, "driving operation corresponding to the visual recognition required point" means the driving operation to be performed by the driver when the driver visually recognizes the visual recognition required point (for example, in the case where the visual recognition required point is a red light, the driving operation corresponding thereto is the decelerating operation), is set in advance for each of the visual recognition required points, and is stored in the memory 10b or the like.

From a result of the visual recognition determination that is based on whether the driver's sightline or eye gaze is directed within the specified range including the visual recognition required point, the controller 10 calculates a ratio (a missing ratio) of the number of the visual recognition required points that are not visually recognized by the driver to the total number of the visual recognition required points (CALCULATE MISSING RATIO). In other words, a missing ratio Fr can be calculated by the following equation.

Missing ratio Fr = (the number of the visual recognition required points not visually recognized by the driver)/(the total number of the visual recognition required points)

Then, when the missing ratio Fr is equal to or higher than a specified threshold A (for example, 40% or higher), it is determined that the driver is in the abnormality sign state. That is, the driver's abnormality sign state is detected (DETECT ABNORMALITY SIGN). This threshold A is set and stored in the memory 10b or the like in advance.

The controller 10 estimates the size of the effective visual field from the result of the visual recognition determination that is based on whether the driving operation corresponding to the visual recognition required point is performed when the driver's eye gaze is directed within the specified range including the visual recognition required point (ESTIMATE EFFECTIVE VISUAL FIELD). More specifically, the controller 10 acquires a minimum value (a minimum outside effective visual field angle) of the angles, each of which is defined by the direction from the driver's head toward the visual recognition required point and the driver's eye gaze, in regard to the visual recognition required points, each of which is determined not to be visually recognized by the driver. The controller 10 also acquires a maximum value (a maximum inside effective visual field angle) of the angles, each of which is defined by the direction from the driver's head toward the visual recognition required point and the driver's eye gaze, in regard to the visual recognition required points, each of which is determined to be visually recognized by the driver. Then, an intermediate value between the minimum outside effective visual field angle and the maximum inside effective visual field angle is defined as an effective visual field size (an effective viewing angle) Fv.

Then, when the effective visual field size Fv is smaller than a specified threshold B (for example, smaller than five degrees), it is determined that the driver is in the abnormality sign state. That is, the driver's abnormality sign state is detected (DETECT ABNORMALITY SIGN). This threshold B is set and stored in the memory 10b or the like in advance.

When detecting the abnormality sign state, the controller 10 transmits, to the PCM 33, the DSC 34, and the EPS 35, the control signal for appropriately actuating the drive power source 2, the transmission 3, the brake 4, and the steering device 5, and transmits, to the display 36 and the speaker 37, the control signal for outputting the desired information. For example, the controller 10 causes the display 36 to display the image information that is used to guide the driver's sightline or eye gaze to the visual recognition required point not visually recognized by the driver, and causes the speaker 37 to output the voice information.

### [Abnormality Sign Detection Processing]

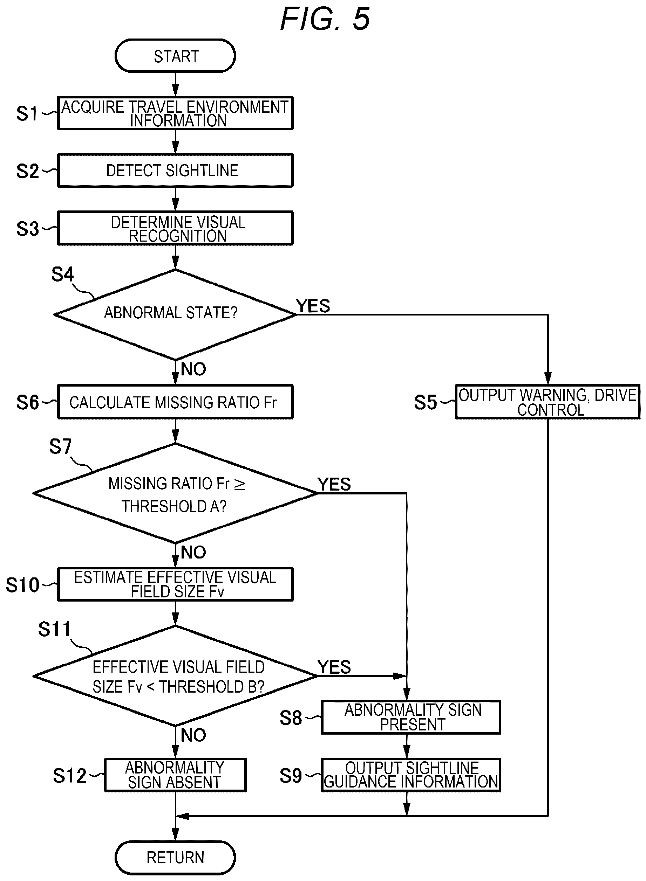

Next, a description will be made on a flow of detection processing of the abnormality sign state by the driver abnormality sign detection apparatus 100 in this embodiment with reference to Fig. 5. Fig. 5 is a flowchart of abnormality sign detection processing.

The abnormality sign detection processing in Fig. 5 is initiated when a power supply of the vehicle 1 is turned on, and is repeatedly executed by the controller 10 at a specified time interval (for example, every 0.05 to 0.2 second).

When the abnormality sign detection processing is initiated, first, the controller 10 acquires the travel environment information on the basis of the signals that are received from the sensors including the outside camera 21, the radar 22, the navigation system 23, and the positioning system 24 (step S1).

In addition, the controller 10 detects the driver's eye gaze on the basis of the signal that is received from the in-vehicle camera 32 (step S2). Then, the controller 10 identifies the visual recognition required point to be visually recognized by the driver on the basis of the travel environment information that is acquired in step S1, and determines whether the driver has visually recognized the visual recognition required point on the basis of the identified visual recognition required point and the driver's eye gaze that is detected in step S2 (step S3). As described above, in the case where the driver's sightline or eye gaze is directed within the specified range including the visual recognition required point, the controller 10 determines that the driver has visually recognized the visual recognition required point. In addition, in the case where the driver's eye gaze is directed within the specified range including the visual recognition required point, and thereafter the driving operation corresponding to the visual recognition required point is performed, the controller 10 determines that the driver has visually recognized the visual recognition required point.

Next, the controller 10 determines whether the driver is in the abnormal state on the basis of the signals that are received from the sensors including the outside camera 21, the radar 22, the navigation system 23, the positioning system 24, the vehicle speed sensor 25, the acceleration sensor 26, the yaw rate sensor 27, the steering angle sensor 28, the steering torque sensor 29, the acceleration sensor 30, the brake sensor 31, and the in-vehicle camera 32 (step S4).

For example, as in the technique disclosed in JP-A-2021-077136, the controller 10 detects an amplitude and a frequency of the vehicle driver's saccade, detects such an attention level that is increased as the number of caution points to be checked by the driver during the travel is increased in the external environment of the vehicle, and determines whether the driver is in the abnormal state on the basis of the attention level and the amplitude and the frequency of the driver's saccade. Alternatively, the controller 10 detects the driver's sightline or gaze direction, a posture (a position of an upper body or the head), opening amounts of the driver's eye lids, the driver's steering wheel grip force, or the like. In this way, based on such a detection result, the controller 10 can also determine that the driver is in the abnormal state. For example, in the case where stability of the eye gaze direction is lower than a specified value, in the case where stability of the posture is lower than a specified value, in the case where the eye lids keep closed for a specified time or longer, or in the case where the steering wheel grip force is smaller than a specified value, the controller 10 can determine that the driver is in the abnormal state. In addition, for example, in the case where stability of the position of the vehicle 1 from a center line on the travel road, stability of the steering angle, or the like is lower than a specified value, the controller 10 can estimate that the driver is in the abnormal state.

As a result, if it is determined that the driver is in the abnormal state (step S4: YES), the controller 10 transmits the control signal to the display 36 and the speaker 37 and causes the display 36 and the speaker 37 to output a warning. The controller 10 also transmits, to the PCM 33, the DSC 34, and the EPS 35, the control signal to appropriately actuate the drive power source 2, the transmission 3, the brake 4, and the steering device 5, and thereby controls the behavior of the vehicle 1 such that the vehicle 1 is safely stopped on a road shoulder, for example (step S5). After step S5, the controller 10 terminates the abnormality sign detection processing.

On the other hand, if it is determined that the driver is not in the abnormal state (step S4: NO), the controller 10 calculates the missing ratio Fr from the result of the visual recognition determination that is based on whether the driver's eye gaze is directed within the specified range including the visual recognition required point (step S6).

Next, the controller 10 determines whether the missing ratio Fr calculated in step S6 is equal to or higher than the specified threshold A (step S7). As a result, if the missing ratio Fr is equal to or higher than the specified threshold A (step S7: YES), the controller 10 determines that the driver is in the abnormality sign state. That is, the driver's abnormality sign state is detected (step S8).

Next, the controller 10 transmits the control signal to the display 36 and the speaker 37, and causes the display 36 and the speaker 37 to respectively output the image information and the voice information (sightline guidance information) that are used to guide the driver's eye gaze to the visual recognition required point not visually recognized by the driver (step S9). After step S9, the controller 10 terminates the abnormality sign detection processing.

On the other hand, in step S7, if the missing ratio Fr is not equal to or higher than the specified threshold A (is lower than the threshold A) (step S7: NO), the controller 10 estimates the effective visual field size Fv from the result of the visual recognition determination in step S3 that is based on whether the driving operation corresponding to the visual recognition required point is performed when the driver's sightline or eye gaze is directed within the specified range including the visual recognition required point (step S10).

Next, the controller 10 determines whether the effective visual field size Fv estimated in step S10 is smaller than the specified threshold B (step S11). As a result, if the effective visual field size Fv is smaller than the specified threshold B (step S11: YES), the controller 10 determines that the driver is in the abnormality sign state. That is, the driver's abnormality sign state is detected (step S8). In this case, the controller 10 causes the display 36 and the speaker 37 to output the sightline guidance information (step S9), and terminates the abnormality sign detection processing.

On the other hand, in step S11, if the effective visual field size Fv is not smaller than the specified threshold B (is equal to or larger than the threshold B) (step S11: NO), the controller 10 determines that the driver is not in the abnormality sign state. That is, the driver's abnormality sign state is not detected (step S12). After step S12, the controller 10 terminates the abnormality sign detection processing.

In the abnormality sign detection processing in this embodiment, the controller 10 detects the driver's abnormality sign state on the basis of each of whether the missing ratio Fr is equal to or higher than the specified threshold A and whether the effective visual field size Fv is smaller than the specified threshold B. However, the controller 10 may detect the abnormality sign state on the basis of only one of the missing ratio Fr and the effective visual field size Fv.

In addition, in the abnormality sign detection processing in this embodiment, the controller 10 detects the driver's abnormality sign state when determining in step S4 that the driver is not in the abnormal state (step S4: NO). However, the determination on whether the driver is in the abnormal state may not be made, or the determination on whether the driver is in the abnormal state may be made after the abnormality sign state is detected.

Furthermore, in this embodiment, the effective visual field size Fv is estimated from the result of the visual recognition determination that is based on whether the driving operation corresponding to the visual recognition required point is performed when the driver's eye gaze is directed within the specified range including the visual recognition required point. However, the effective visual field size may be estimated by a different method therefrom. For example, the effective visual field size can also be estimated on the basis of an angle that is defined by a direction from the driver's head toward the object with high saliency (for example, the object with a significant color difference or luminance difference from the surrounding area, or the object with large motion with respective to the surroundings) and the driver's eye gaze at the time when the driver's eye gaze is directed to such an object.

### [Operational Effects]

Next, a description will be made on operational effects of the driver abnormality sign detection apparatus 100 in the above-described embodiment.

The controller 10 identifies the visual recognition required point to be visually recognized by the driver on the basis of the travel environment information, determines whether the driver has visually recognized the visual recognition required point on the basis of the driver's eye gaze, and detects the driver's abnormality sign state on the basis of the fact that the driver has not visually recognized the visual recognition required point. Thus, it is possible to detect the abnormality sign state by using the fact that the driver has not been able to visually recognize the visual recognition required point due to the decline in the distributive attention function or a reduction in the effective visual field at the initial stage of the decline in the driver's attention function. In this way, it is possible to detect the driver's abnormality sign state at a stage, at which no obvious difference appears to the amplitude and the frequency of the driver's saccade and which is sufficiently earlier than onset of the abnormal state where the driver has difficulty in driving, for example.

The controller 10 determines that the driver has visually recognized the visual recognition required point in the case where the driver's eye gaze is directed within the specified range including the visual recognition required point, and detects the driver's abnormality sign state on the basis of the number of the visual recognition required points not visually recognized by the driver. Accordingly, when the driver can no longer visually recognize the plural visual recognition required points at the same time due to the decline in the distributive attention function and starts missing the visual recognition required points at the initial stage of the decline in the attention function, the abnormality sign state can be detected on the basis of the severity of missing of the visual recognition required point. In this way, it is possible to appropriately detect the driver's abnormality sign state at the stage sufficiently earlier than onset of the abnormal state where the driver has difficulty in driving.

The controller 10 is configured to detect the driver's abnormality sign state in the case where the missing ratio of the visual recognition required point is equal to or higher than the specified value. Accordingly, when the driver can no longer visually recognize the plural visual recognition required points at the same time due to the decline in the distributive attention function and the missing ratio of the visual recognition required point becomes equal to or higher than the specified value at the initial stage of the decline in the attention function, the abnormality sign state can be detected. In this way, it is possible to appropriately detect the driver's abnormality sign state at the stage sufficiently earlier than the onset of the abnormal state where the driver has difficulty in driving.

The controller 10 estimates the effective visual field size that can be visually recognized by the driver on the basis of the distance between the driver's eye gaze and the visual recognition required point not visually recognized by the driver. Then, in the case where the estimated effective visual field size is smaller than the specified value, the driver's abnormality sign state is detected. Accordingly, when the effective visual field is narrowed and the driver starts missing the visual recognition required point at the initial stage of the decline in the attention function, the abnormality sign state can be detected by estimating the degree of the narrowness of the effective visual field. In this way, it is possible to appropriately detect the driver's abnormality sign state at the stage sufficiently earlier than the onset of the abnormal state where the driver has difficulty in driving.

The driver abnormality sign detection apparatus 100 further includes the display 36 and the speaker 37, each of which outputs the information to the driver. When detecting the driver's abnormality sign state, the controller 10 causes the display 36 and the speaker 37 to output the information to guide the driver's sightline or eye gaze to the visual recognition required point. Accordingly, in the case where the driver's abnormality sign state is detected, the driver's eye gaze can be guided to the visual recognition required point. Therefore, it is possible to provide the appropriate driver assistance to the driver who starts missing the visual recognition required point at the initial stage of the decline in the attention function.

### [Description of Reference Signs and Numerals]

1 Vehicle
10 Controller
100 Driver abnormality sign detection apparatus
21 Outside camera
22 Radar
23 Navigation system
24 Positioning system
25 Vehicle speed sensor
26 Acceleration sensor
27 Yaw rate sensor
28 Steering angle sensor
29 Steering torque sensor
30 Acceleration sensor
31 Brake sensor
32 In-vehicle camera
36 Display
37 Speaker

## Claims

1. A driver abnormality sign detection apparatus (100) that is adapted to detect an abnormality sign state of a driver who drives a vehicle (1), the driver abnormality sign detection apparatus (100) comprising:
a travel environment information acquisition device (21-24) for acquiring travel environment information of the vehicle (1);
an eye gaze tracker (32) for detecting the driver's eye gaze; and
a controller (10) configured to detect the driver's abnormality sign state on the basis of the travel environment information and the driver's eye gaze, wherein
the controller (10) is configured to:
identify a visual recognition required point to be visually recognized by the driver on the basis of the travel environment information;
determine whether the driver has visually recognized the visual recognition required point on the basis of the driver's eye gaze; and
detect the driver's abnormality sign state on the basis of a fact that the driver has not visually recognized the visual recognition required point, **characterized in that**
the driver abnormality sign detection apparatus (100) further comprises:
a driving operation detector (25-31) for detecting a driving operation of the vehicle (1) caused by the driver, wherein
the controller (10) is adapted to determine that the driver has visually recognized the visual recognition required point, in the case where the driver's eye gaze is directed within a specified range including the visual recognition required point, and, thereafter, the driving operation performed by the driver of the vehicle corresponds to the visual recognition required point.

2. The driver abnormality sign detection apparatus (100) according to claim 1 wherein
the controller (10) is configured to:
detect the driver's abnormality sign state on the basis of the number of the visual recognition required point not visually recognized by the driver.

3. The driver abnormality sign detection apparatus (100) according to any one of the preceding claims, wherein
the controller (10) is configured to detect the driver's abnormality sign state in the case where a ratio (Fr) of the number of the visual recognition required points not visually recognized by the driver to the total number of the visual recognition required points is equal to or larger than a specified value.

4. The driver abnormality sign detection apparatus (100) according to any one of the preceding claims, wherein
the controller (10) is configured to:
estimate a size of an effective visual field that can be visually recognized by the driver on the basis of a distance between the driver's eye gaze and the visual recognition required point not visually recognized by the driver; and
detect the driver's abnormality sign state in the case where the estimated effective visual field size is smaller than a specified value.

5. The driver abnormality sign detection apparatus (100) according to the preceding claim 4**,** wherein the controller is configured to estimate the size of the effective visual field by comparing a minimum value of the angles of the driver's head with respect to at least one visual recognition required point that has not been visually recognized and the driver's eye gaze with a maximum value of the angles of the driver's head with respect to at least one visual recognition required point that has been visually recognized and the driver's eye gaze.

6. The driver abnormality sign detection apparatus (100) according to one of the preceding claims 4 or 5, wherein the controller is configured to estimate the size of the effective visual field by being an intermediate value, preferably a mean value, between the minimum value of the angles of the driver's head with respect to at least one visual recognition required point that has not been visually recognized and the driver's eye gaze and the maximum value of the angles of the driver's head with respect to at least one visual recognition required point that has been visually recognized and the driver's eye gaze.

7. The driver abnormality sign detection apparatus (100) according to any one of the preceding claims further comprising:
an information output device configured to output information to the driver, wherein
the controller (10) is configured to cause the information output device 35, 36 to output information that is used to guide the driver's eye gaze to the visual recognition required point in the case where the driver's abnormality sign state is detected.

8. The driver abnormality sign detection apparatus (100) according to any one of the preceding claims, wherein the travel environment information acquisition device (21-24) for acquiring travel environment information of the vehicle (1) comprises at least one of: an outside camera (21) for capturing an image around the vehicle (1), a radar (22) for measuring a position and a speed of an object with respect to the vehicle (1), a navigation system (23) for providing map information to the controller (10), and a speed sensor for detecting a speed of the vehicle (1).

9. The driver abnormality sign detection apparatus (100) according to any one of the preceding claims, wherein the eye gaze tracker comprises an in-vehicle camera for capturing an image of the driver of the vehicle (1).

10. The driver abnormality sign detection apparatus (100) according to any one of the preceding claims,
wherein
the controller (10) is adapted to determine that the driver has not visually recognized the visual recognition required point, in the case where the driver's eye gaze is directed within a specified range including the visual recognition required point, and, thereafter, the driving operation performed by the driver of the vehicle fails to correspond to the visual recognition required point.

11. The driver abnormality sign detection apparatus (100) according to one of the preceding claims 9 or 10, wherein the driving operation detector (25-31) comprises at least one of: a vehicle speed sensor (25) for detecting a speed of the vehicle (1), a yaw rate sensor (27) for detecting a yaw rate of the vehicle (1), a steering angle sensor (28) for detecting a rotation angle of a steering wheel (5), a steering torque sensor (29) for detecting torque applied to a steering shaft via a steering wheel (5), an acceleration sensor (26, 30) for detecting an depression amount of an accelerator pedal, and a brake sensor (31) for detecting a depression amount of a brake pedal.

12. The driver abnormality sign detection apparatus (100) according to one of the preceding claims 7 to 9, wherein the visual recognition required point is a red light or a stop sign, the driving operation corresponding thereto is the decelerating operation.

13. The driver abnormality sign detection apparatus (100) according to any one of the preceding claims, further comprising a visual recognition required point database in which the travel environment and the visual recognition required points are stored in a mutually corresponding manner, for identifying the visual recognition required points to be visually recognized by the driver on basis of the current travel environment detected by the travel environment information acquisition device.

14. Vehicle comprising the driver abnormality sign detection apparatus of any one of the preceding claims.

## Patentansprüche

1. Fahrer-Anomalieanzeichen-Erfassungsvorrichtung (100), die dazu eingerichtet ist, einen Anomalieanzeichenzustand eines Fahrers zu erfassen, der ein Fahrzeug (1) fährt, wobei die Fahrer-Anomalieanzeichen-Erfassungsvorrichtung (100) umfasst:
eine Fahrumgebungsinformations-Erfassungseinrichtung (21-24) zum Erfassen von Fahrumgebungsinformationen des Fahrzeugs (1);
einen Blickverfolgungssensor (32) zum Erfassen des Blicks des Fahrers; und
eine Steuereinrichtung (10), die dazu konfiguriert ist, den Anomalieanzeichenzustand des Fahrers auf Grundlage der Fahrumgebungsinformationen und des Blicks des Fahrers zu erfassen, wobei
die Steuereinrichtung (10) dazu konfiguriert ist:
einen visuell zu erkennenden Punkt, der durch den Fahrer visuell zu erkennen ist, auf Grundlage der Fahrumgebungsinformationen zu identifizieren;
auf Grundlage des Blicks des Fahrers zu bestimmen, ob der Fahrer den visuell zu erkennenden Punkt visuell erkannt hat; und
den Anomalieanzeichenzustand des Fahrers auf Grundlage der Tatsache zu erfassen, dass der Fahrer den visuell zu erkennenden Punkt nicht visuell erkannt hat, **dadurch gekennzeichnet, dass**
die Fahrer-Anomalieanzeichen-Erfassungsvorrichtung (100) ferner umfasst:
einen Fahrbedienungsdetektor (25-31) zum Erfassen einer durch den Fahrer verursachten Fahrbedienung des Fahrzeugs (1), wobei
die Steuereinrichtung (10) dazu eingerichtet ist, zu bestimmen, dass der Fahrer den visuell zu erkennenden Punkt visuell erkannt hat, wenn der Blick des Fahrers innerhalb eines festgelegten Bereichs gerichtet ist, der den visuell zu erkennenden Punkt einschließt, und anschließend die durch den Fahrer des Fahrzeugs ausgeführte Fahrbedienung dem visuell zu erkennenden Punkt entspricht.

2. Fahrer-Anomalieanzeichen-Erfassungsvorrichtung (100) nach Anspruch 1, wobei die Steuereinrichtung (10) dazu konfiguriert ist:
den Anomalieanzeichenzustand des Fahrers auf Grundlage der Anzahl der durch den Fahrer nicht visuell erkannten visuell zu erkennenden Punkte zu erfassen.

3. Fahrer-Anomalieanzeichen-Erfassungsvorrichtung (100) nach einem der vorstehenden Ansprüche, wobei die Steuereinrichtung (10) dazu konfiguriert ist, den Anomalieanzeichenzustand des Fahrers zu erfassen, wenn ein Verhältnis (Fr) der Anzahl der durch den Fahrer nicht visuell erkannten visuell zu erkennenden Punkte zur Gesamtanzahl der visuell zu erkennenden Punkte gleich einem festgelegten Wert oder größer als dieser ist.

4. Fahrer-Anomalieanzeichen-Erfassungsvorrichtung (100) nach einem der vorstehenden Ansprüche, wobei
die Steuereinrichtung (10) dazu konfiguriert ist:
eine Größe eines wirksamen Sichtfelds, das durch den Fahrer visuell erkannt werden kann, auf Grundlage eines Abstands zwischen dem Blick des Fahrers und dem durch den Fahrer nicht visuell erkannten visuell zu erkennenden Punkt abzuschätzen; und
den Anomalieanzeichenzustand des Fahrers zu erfassen, wenn die abgeschätzte Größe des wirksamen Sichtfelds kleiner als ein festgelegter Wert ist.

5. Fahrer-Anomalieanzeichen-Erfassungsvorrichtung (100) nach dem vorstehenden Anspruch 4, wobei die Steuereinrichtung dazu konfiguriert ist, die Größe des wirksamen Sichtfelds abzuschätzen, indem ein Minimalwert der Winkel des Kopfes des Fahrers in Bezug auf mindestens einen visuell zu erkennenden Punkt, der nicht visuell erkannt worden ist, und den Blick des Fahrers mit einem Maximalwert der Winkel des Kopfes des Fahrers in Bezug auf mindestens einen visuell zu erkennenden Punkt, der visuell erkannt worden ist, und den Blick des Fahrers verglichen wird.

6. Fahrer-Anomalieanzeichen-Erfassungsvorrichtung (100) nach einem der vorstehenden Ansprüche 4 oder 5, wobei die Steuereinrichtung dazu konfiguriert ist, die Größe des wirksamen Sichtfelds dadurch abzuschätzen, dass sie ein Zwischenwert, vorzugsweise ein Mittelwert, zwischen dem Minimalwert der Winkel des Kopfes des Fahrers in Bezug auf mindestens einen visuell zu erkennenden Punkt, der nicht visuell erkannt worden ist, und den Blick des Fahrers und dem Maximalwert der Winkel des Kopfes des Fahrers in Bezug auf mindestens einen visuell zu erkennenden Punkt, der visuell erkannt worden ist, und den Blick des Fahrers ist.

7. Fahrer-Anomalieanzeichen-Erfassungsvorrichtung (100) nach einem der vorstehenden Ansprüche, ferner umfassend:
eine Informationsausgabeeinrichtung, die dazu konfiguriert ist, Informationen an den Fahrer auszugeben, wobei die Steuereinrichtung (10) dazu konfiguriert ist, die Informationsausgabeeinrichtung 35, 36 zu veranlassen, Informationen auszugeben, die dazu verwendet werden, den Blick des Fahrers zu dem visuell zu erkennenden Punkt zu führen, wenn der Anomalieanzeichenzustand des Fahrers erfasst wird.

8. Fahrer-Anomalieanzeichen-Erfassungsvorrichtung (100) nach einem der vorstehenden Ansprüche, wobei die Fahrumgebungsinformations-Erfassungseinrichtung (21-24) zum Erfassen von Fahrumgebungsinformationen des Fahrzeugs (1) mindestens eines umfasst von: einer Außenkamera (21) zum Aufnehmen eines Bildes um das Fahrzeug (1) herum, einem Radar (22) zum Messen einer Position und einer Geschwindigkeit eines Objekts in Bezug auf das Fahrzeug (1), einem Navigationssystem (23) zum Bereitstellen von Karteninformationen an die Steuereinrichtung (10) und einem Geschwindigkeitssensor zum Erfassen einer Geschwindigkeit des Fahrzeugs (1).

9. Fahrer-Anomalieanzeichen-Erfassungsvorrichtung (100) nach einem der vorstehenden Ansprüche, wobei der Blickverfolgungssensor eine fahrzeuginterne Kamera zum Aufnehmen eines Bildes des Fahrers des Fahrzeugs (1) umfasst.

10. Fahrer-Anomalieanzeichen-Erfassungsvorrichtung (100) nach einem der vorstehenden Ansprüche,
wobei die Steuereinrichtung (10) dazu eingerichtet ist, zu bestimmen, dass der Fahrer den visuell zu erkennenden Punkt nicht visuell erkannt hat, wenn der Blick des Fahrers innerhalb eines festgelegten Bereichs gerichtet ist, der den visuell zu erkennenden Punkt einschließt, und anschließend die durch den Fahrer des Fahrzeugs ausgeführte Fahrbedienung dem visuell zu erkennenden Punkt nicht entspricht.

11. Fahrer-Anomalieanzeichen-Erfassungsvorrichtung (100) nach einem der vorstehenden Ansprüche 9 oder 10, wobei der Fahrbedienungsdetektor (25-31) mindestens eines umfasst von: einem Fahrzeuggeschwindigkeitssensor (25) zum Erfassen einer Geschwindigkeit des Fahrzeugs (1), einem Gierratensensor (27) zum Erfassen einer Gierrate des Fahrzeugs (1), einem Lenkwinkelsensor (28) zum Erfassen eines Drehwinkels eines Lenkrads (5), einem Lenkmomentsensor (29) zum Erfassen eines Drehmoments, das über ein Lenkrad (5) auf eine Lenkwelle ausgeübt wird, einem Beschleunigungssensor (26, 30) zum Erfassen eines Betätigungswegs eines Gaspedals und einem Bremssensor (31) zum Erfassen eines Betätigungswegs eines Bremspedals.

12. Fahrer-Anomalieanzeichen-Erfassungsvorrichtung (100) nach einem der vorstehenden Ansprüche 7 bis 9, wobei der visuell zu erkennende Punkt eine rote Ampel oder ein Stoppschild ist und die diesem entsprechende Fahrbedienung die Verzögerungsbedienung ist.

13. Fahrer-Anomalieanzeichen-Erfassungsvorrichtung (100) nach einem der vorstehenden Ansprüche, ferner umfassend eine Datenbank für visuell zu erkennende Punkte, in der die Fahrumgebung und die visuell zu erkennenden Punkte einander entsprechend gespeichert sind, zum Identifizieren der durch den Fahrer visuell zu erkennenden Punkte auf Grundlage der aktuellen Fahrumgebung, die durch die Fahrumgebungsinformations-Erfassungseinrichtung erfasst wird.

14. Fahrzeug, umfassend die Fahrer-Anomalieanzeichen-Erfassungsvorrichtung nach einem der vorstehenden Ansprüche.

## Revendications

1. Appareil de détection de signe d'anomalie du conducteur (100), qui est adapté pour détecter un état de signe d'anomalie d'un conducteur qui conduit un véhicule (1), l'appareil de détection de signe d'anomalie du conducteur (100) comprenant :
un dispositif d'acquisition d'informations d'environnement de conduite (21-24) pour acquérir des informations d'environnement de conduite du véhicule (1) ;
un dispositif de suivi du regard (32) pour détecter le regard du conducteur ; et
un contrôleur (10) configuré pour détecter l'état de signe d'anomalie du conducteur sur la base des informations d'environnement de conduite et du regard du conducteur, dans lequel
le contrôleur (10) est configuré pour :
identifier un point nécessitant une reconnaissance visuelle devant être reconnu visuellement par le conducteur sur la base des informations d'environnement de conduite ;
déterminer si le conducteur a reconnu visuellement le point nécessitant une reconnaissance visuelle sur la base du regard du conducteur ; et
détecter l'état de signe d'anomalie du conducteur sur la base du fait que le conducteur n'a pas reconnu visuellement le point nécessitant une reconnaissance visuelle, **caractérisé en ce que**
l'appareil de détection de signe d'anomalie du conducteur (100) comprend en outre :
un détecteur d'opération de conduite (25-31) pour détecter une opération de conduite du véhicule (1) causée par le conducteur, dans lequel
le contrôleur (10) est adapté pour déterminer que le conducteur a reconnu visuellement le point nécessitant une reconnaissance visuelle, dans le cas où le regard du conducteur est dirigé dans une plage spécifiée incluant le point nécessitant une reconnaissance visuelle, et, par la suite, l'opération de conduite effectuée par le conducteur du véhicule correspond au point nécessitant une reconnaissance visuelle.

2. Appareil de détection de signe d'anomalie du conducteur (100) selon la revendication 1, dans lequel le contrôleur (10) est configuré pour :
détecter l'état de signe d'anomalie du conducteur sur la base du nombre de points nécessitant une reconnaissance visuelle non reconnus visuellement par le conducteur.

3. Appareil de détection de signe d'anomalie du conducteur (100) selon l'une quelconque des revendications précédentes, dans lequel le contrôleur (10) est configuré pour détecter l'état de signe d'anomalie du conducteur dans le cas où un rapport (Fr) du nombre des points nécessitant une reconnaissance visuelle non reconnus visuellement par le conducteur au nombre total des points nécessitant une reconnaissance visuelle est égal ou supérieur à une valeur spécifiée.

4. Appareil de détection de signe d'anomalie du conducteur (100) selon l'une quelconque des revendications précédentes, dans lequel
le contrôleur (10) est configuré pour :
estimer une taille d'un champ visuel effectif qui peut être reconnu visuellement par le conducteur sur la base d'une distance entre le regard du conducteur et le point nécessitant une reconnaissance visuelle non reconnu visuellement par le conducteur ; et
détecter l'état de signe d'anomalie du conducteur dans le cas où la taille estimée du champ visuel effectif est inférieure à une valeur spécifiée.

5. Appareil de détection de signe d'anomalie du conducteur (100) selon la revendication précédente 4, dans lequel le contrôleur est configuré pour estimer la taille du champ visuel effectif en comparant une valeur minimale des angles de la tête du conducteur par rapport à au moins un point nécessitant une reconnaissance visuelle qui n'a pas été reconnu visuellement et au regard du conducteur avec une valeur maximale des angles de la tête du conducteur par rapport à au moins un point nécessitant une reconnaissance visuelle qui a été reconnu visuellement et au regard du conducteur.

6. Appareil de détection de signe d'anomalie du conducteur (100) selon l'une des revendications précédentes 4 ou 5, dans lequel le contrôleur est configuré pour estimer la taille du champ visuel effectif en étant une valeur intermédiaire, de préférence une valeur moyenne, entre la valeur minimale des angles de la tête du conducteur par rapport à au moins un point nécessitant une reconnaissance visuelle qui n'a pas été reconnu visuellement et au regard du conducteur et la valeur maximale des angles de la tête du conducteur par rapport à au moins un point nécessitant une reconnaissance visuelle qui a été reconnu visuellement et au regard du conducteur.

7. Appareil de détection de signe d'anomalie du conducteur (100) selon l'une quelconque des revendications précédentes, comprenant en outre :
un dispositif de sortie d'informations configuré pour sortir des informations au conducteur, dans lequel le contrôleur (10) est configuré pour amener le dispositif de sortie d'informations 35, 36 à sortir des informations qui sont utilisées pour guider le regard du conducteur vers le point nécessitant une reconnaissance visuelle dans le cas où l'état de signe d'anomalie du conducteur est détecté.

8. Appareil de détection de signe d'anomalie du conducteur (100) selon l'une quelconque des revendications précédentes, dans lequel le dispositif d'acquisition d'informations d'environnement de conduite (21-24) pour acquérir des informations d'environnement de conduite du véhicule (1) comprend au moins l'un parmi : une caméra extérieure (21) pour capturer une image autour du véhicule (1), un radar (22) pour mesurer une position et une vitesse d'un objet par rapport au véhicule (1), un système de navigation (23) pour fournir des informations cartographiques au contrôleur (10), et un capteur de vitesse pour détecter une vitesse du véhicule (1).

9. Appareil de détection de signe d'anomalie du conducteur (100) selon l'une quelconque des revendications précédentes, dans lequel le dispositif de suivi du regard comprend une caméra embarquée pour capturer une image du conducteur du véhicule (1).

10. Appareil de détection de signe d'anomalie du conducteur (100) selon l'une quelconque des revendications précédentes,
dans lequel le contrôleur (10) est adapté pour déterminer que le conducteur n'a pas reconnu visuellement le point nécessitant une reconnaissance visuelle, dans le cas où le regard du conducteur est dirigé dans une plage spécifiée incluant le point nécessitant une reconnaissance visuelle, et, par la suite, l'opération de conduite effectuée par le conducteur du véhicule ne parvient pas à correspondre au point nécessitant une reconnaissance visuelle.

11. Appareil de détection de signe d'anomalie du conducteur (100) selon l'une des revendications précédentes 9 ou 10, dans lequel le détecteur d'opération de conduite (25-31) comprend au moins l'un parmi : un capteur de vitesse du véhicule (25) pour détecter une vitesse du véhicule (1), un capteur de vitesse de lacet (27) pour détecter une vitesse de lacet du véhicule (1), un capteur d'angle de braquage (28) pour détecter un angle de rotation d'un volant (5), un capteur de couple de direction (29) pour détecter un couple appliqué à un arbre de direction par l'intermédiaire d'un volant (5), un capteur d'accélération (26, 30) pour détecter une quantité d'enfoncement d'une pédale d'accélérateur, et un capteur de frein (31) pour détecter une quantité d'enfoncement d'une pédale de frein.

12. Appareil de détection de signe d'anomalie du conducteur (100) selon l'une des revendications précédentes 7 à 9, dans lequel le point nécessitant une reconnaissance visuelle est un feu rouge ou un panneau d'arrêt, l'opération de conduite correspondant à celui-ci étant l'opération de décélération.

13. Appareil de détection de signe d'anomalie du conducteur (100) selon l'une quelconque des revendications précédentes, comprenant en outre une base de données de points nécessitant une reconnaissance visuelle dans laquelle l'environnement de conduite et les points nécessitant une reconnaissance visuelle sont stockés d'une manière mutuellement correspondante, pour identifier les points nécessitant une reconnaissance visuelle devant être reconnus visuellement par le conducteur sur la base de l'environnement de conduite actuel détecté par le dispositif d'acquisition d'informations d'environnement de conduite.

14. Véhicule comprenant l'appareil de détection de signe d'anomalie du conducteur selon l'une quelconque des revendications précédentes.
